# EUROPEAN PATENT SPECIFICATION

(11) **EP 3 517 278 B1**
(45) Date of publication and mention of the grant of the patent: **14.04.2021**
(21) Application number: 17883925.4
(22) Date of filing: 15.12.2017
(51) Int. Cl.: B29C 65/08, B29C 59/04, B32B 3/30, B32B 7/08

(54) **COMPOSITE SHEET MANUFACTURING METHOD AND MANUFACTURING APPARATUS**
VERBUNDFOLIENHERSTELLUNGSVERFAHREN UND -HERSTELLUNGSVORRICHTUNG
PROCÉDÉ DE FABRICATION DE FEUILLE COMPOSITE ET APPAREIL DE FABRICATION

(30) Priority: 19.12.2016 JP 2016245886; 12.12.2017 JP 2017237899
(43) Date of publication of application: 31.07.2019
(73) Proprietor: Kao Corporation, Chuo-ku Tokyo 103-8210 (JP)
(72) Inventor: KURODA, Keisuke, Haga-gun Tochigi 321-3497 (JP); MATSUNAGA, Ryuji, Haga-gun Tochigi 321-3497 (JP); MORITA, Shinnosuke, Haga-gun Tochigi 321-3497 (JP); SAITOU, Kazuma, Haga-gun Tochigi 321-3497 (JP)
(74) Representative: Vossius & Partner Patentanwälte Rechtsanwälte mbB
(86) International application number: PCT/JP2017/045200
(87) International publication number: WO 2018/116996

(56) References cited:
- EP-A1- 1 419 754
- WO-A1-2014/024643
- WO-A1-2015/146704
- WO-A1-2018/100901
- JP-A- H1 080 445
- JP-A- 2004 174 234

## Description

### Technical Field

The present invention relates to methods for manufacturing composite sheets and apparatuses for manufacturing composite sheets.

### Background Art

Known absorbent articles, such as disposable diapers and sanitary napkins, include a topsheet having projections and depressions on its side to be brought into contact with a wearer.

The present applicant, for example, has proposed a composite sheet including a first sheet and a second sheet that are fusion-bonded at a large number of fusion bonds, and the first sheet has projections projecting to the opposite side of the second sheet at parts other than the fusion bonds. Such a composite sheet has projections and depressions on the surface, and so can give comfortable feel to the skin while in use and can prevent liquid from spreading.

A composite sheet having through-holes at the fusion bonds is also known to improve the liquid wicking property and the like (see Patent Literatures 1 and 2). Patent Literature 2 describes a technique of forming such fusion bonds having through-holes. To this end, rollers have projections and depressions on their surfaces and have small projections on the top face of the projections to form openings in the composite sheet so that the small projections are different in height from the surrounding shoulder portions. Two sheets of the composite sheet are pinched between these small projections and an anvil roller and are heated to form fusion bonds having openings.

### Citation List

### Patent Literatures

Patent Literature 1 : JP 2006-175688 A
Patent Literature 2 : JP 2006-175689 A

### Summary of Invention

The present invention provides a method for manufacturing a composite sheet including a large number of fusion bonds where a first sheet and a second sheet are fusion-bonded, the first sheet having projections projecting to the opposite side of the second sheet at least partially at parts other than the fusion bonds. The method includes an embossing step of introducing the first sheet to an engaging part of a first roller and a second roller each having projections and depressions engaging with each other on the peripheral surfaces while rotating both of the first and second rollers to deform the first sheet to have depressions and projections; an overlapping step of conveying the deformed first sheet having depressions and projections while holding the first sheet on the first roller, and overlapping the second sheet with the first sheet being conveyed; and an ultrasonic processing step of pinching the overlapped first and second sheets between the projections of the first roller and an ultrasonic horn of an ultrasonic bonding machine to apply ultrasonic vibrations to the sheets. At the ultrasonic processing step, the fusion bonds having through-holes are formed during application of the ultrasonic vibrations.

The present invention also provides an apparatus for manufacturing a composite sheet including a large number of fusion bonds where a first sheet and a second sheet are fusion-bonded, the first sheet having projections projecting to the opposite side of the second sheet at least partially at parts other than the fusion bonds. The apparatus includes an embossing unit including a first roller and a second roller each having projections and depressions engaging with each other on peripheral surfaces, the embossing unit being configured to deform the first sheet introduced to an engagement part of the first and second rollers to have depressions and projections; and an ultrasonic processing unit including an ultrasonic bonding machine and being configured to overlap the second sheet with the deformed first sheet having depressions and projections and pinch the overlapped first and second sheets between the projections of the first roller and an ultrasonic horn of the ultrasonic bonding machine to partially apply ultrasonic vibrations to the sheets and form the fusion bonds having through-holes.

### Brief Description of Drawings

[Fig. 1] Fig. 1 is a perspective view of a major part of a composite sheet as one example that is manufactured by a method or apparatus for manufacturing a composite sheet of the present invention.
[Fig. 2] Fig. 2 is an enlarged plan view of the composite sheet of Fig. 1 viewed from the side of the first sheet.
[Fig. 3] Fig. 3 schematically shows a method for manufacturing a composite sheet of the present invention that is a first embodiment and an apparatus for manufacturing a composite sheet of the present invention that is a first embodiment.
[Fig. 4] Fig. 4 is an enlarged perspective view of a major part of the first roller of Fig. 3.
[Fig. 5] Fig. 5 shows a major part of the ultrasonic bonding machine of Fig. 3 viewed from the left of Fig. 3.
[Fig. 6] Fig. 6 shows a method for manufacturing a composite sheet of the present invention that is a second embodiment and a major part of an apparatus for manufacturing a composite sheet of the present invention that is a second embodiment.
[Fig. 7] Fig. 7 shows a method for manufacturing a composite sheet of the present invention that is a third embodiment and a major part of an apparatus for manufacturing a composite sheet of the present invention that is a third embodiment.
[Fig. 8] Fig. 8(a) shows another example of the ultrasonic horn including a synthetic resin layer having a heat-storage property at a distal-end part, and Fig. 8(b) shows a state where only a connecting layer is formed by thermal spraying at the distal-end face of the body of the ultrasonic horn made of metal, such as titanium alloy.

### Description of Embodiments

When fusion bonds and through-holes of a composite sheet are separately formed during the manufacture of a composite sheet, the resultant fusion bonds and through-holes may be displaced. The method described in Patent Literature 2 for providing small projections at the top face of the projections also leaves room for improvement because small projections at the top face of the projections easily wear and so the burden for maintenance is large.

The present invention therefore relates to a method for manufacturing a composite sheet and an apparatus for manufacturing a composite sheet that can solve the problems of the conventional techniques.

Referring to the drawings, the following describes the present invention based on its preferred embodiments.

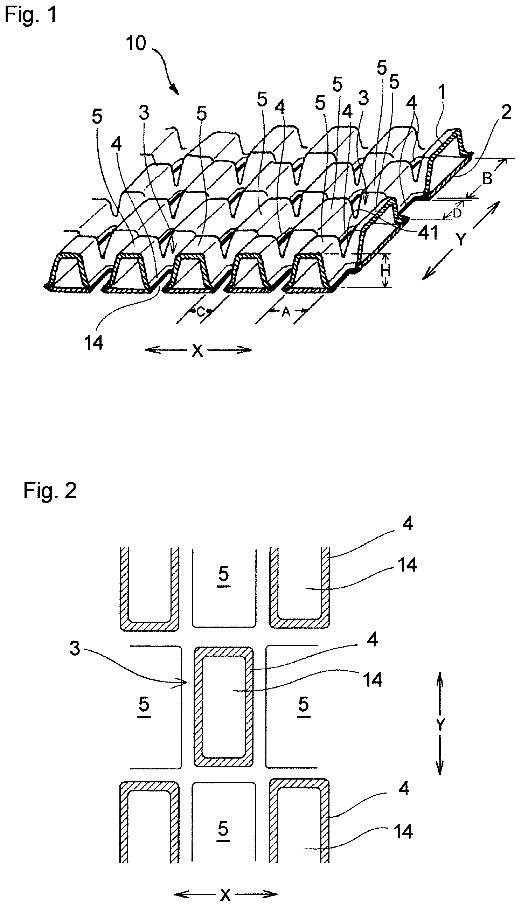

Referring firstly to Fig. 1, the following describes a composite sheet that is manufactured by the method for manufacturing a composite sheet or an apparatus for manufacturing a composite sheet of the present invention.

A composite sheet 10 of Fig. 1 is one example of a composite sheet manufactured by a method for manufacturing a composite sheet or an apparatus for manufacturing a composite sheet of the present invention. As shown in Fig. 1, the composite sheet includes a large number of fusion bonds 4 where a first sheet 1 and a second sheet 2 are fusion-bonded, and at least a part of the first sheet 1 other than the fusion bonds 4 forms projections 5 projecting to the opposite side of the second sheet 2.

The composite sheet 10 is favorably used for a topsheet of an absorbent article, for example. For use as the topsheet of an absorbent article, the first sheet 1 forms one of surfaces of the topsheet that faces the skin of a wearer (hereinafter referred to as skin facing side), while the second sheet 2 forms the other surface that faces an absorbent member (non-skin facing side) while worn.

The projections 5 and the fusion bonds 4 are arranged alternately to make a line in direction X of Fig. 1 that is one direction parallel to the plane of the composite sheet 10. Such lines of the alternating projections and fusion bonds are arrayed in multiple rows in direction Y of Fig. 1 that is the direction parallel to the plane of the composite sheet 10 and is orthogonal to the one direction. The projections 5 and fusion bonds 4 of adjacent lines are respectively out of alignment in direction X. More specifically, their positions are displaced by a half of the pitch in direction X.

Direction Y of the composite sheet 10 corresponds to the sheet-flowing direction (MD, machine direction) during manufacturing, and direction X corresponds to the direction (CD) orthogonal to the sheet-flowing direction during manufacturing.

The first sheet 1 and the second sheet 2 each include a sheet material. For the sheet material, fiber sheets, such as nonwoven fabric, woven fabric and knitted fabric, and films may be used. Among them, a fiber sheet is preferably used because of the comfortable feel to the skin, for example, and nonwoven fabric is used more preferably. The sheet materials of the first sheet 1 and the second sheet 2 may be of the same type or of different types.

When nonwoven fabric is used for the sheet materials of the first sheet 1 and the second sheet 2, examples of the nonwoven fabric include air-through nonwoven fabric, spunbond nonwoven fabric, spunlace nonwoven fabric, melt-blown nonwoven fabric, resin-bond nonwoven fabric, and needle-punch nonwoven fabric. A laminate including two types or more of these nonwoven fabrics or a laminate including the combination of these nonwoven fabrics and a film also may be used, for example. The weight per unit area of nonwoven fabric used for the sheet materials of the first sheet 1 and the second sheet 2 is preferably 10 g/m² or more and more preferably 15 g/m² or more, or is preferably 40 g/m² or less and more preferably 35 g/m² or less. The weight per unit area of nonwoven fabric is preferably 10 g/m² or more and 40 g/m² or less, and is more preferably 15 g/m² or more and 35 g/m² or less.

Fibers of nonwoven fabric may include various types of thermoplastic resins. Preferably a sheet material other than nonwoven fabric also includes fibers and resins made of various types of thermoplastic resins.

Thermoplastic resins include polyolefins such as polyethylene, polypropylene and polybutene, polyesters such as polyethylene terephthalate and polybutylene terephthalate, polyamides such as nylon 6 and nylon 66, polyacrylic acid, alkyl ester polymethacrylate, polyvinyl chloride and polyvinylidene chloride. One type of these resins alone or blended resin of two or more types of these resins may be used. Composite fibers of core-in-sheath or side-by-side configuration also may be used.

As shown in Fig. 1, the composite sheet 10 has a large number of depressions 3 surrounded by projections 5 in both of direction X and direction Y on its face of the first sheet 1. Each of the depressions 3 includes the fusion bond 4 at the bottom, and the fusion bond has a through-hole 14. When viewed as a whole, the composite sheet 10 is uneven on the face of the first sheet 1 that has the depressions 3 and the projections 5 and is undulating, and on the face of the second sheet 2, the composite sheet 10 is flat or is a substantially flat face that is less undulating than the face of the first sheet 1.

As shown in Fig. 2, each of the fusion bonds 4 of the composite sheet 10 has a substantially rectangular shape in plan view that is oblong in direction Y, and internally includes the through-hole 14 that has a substantially rectangular shape in plan view. In other words, each of the fusion bonds 4 has an annular shape surrounding the corresponding through-hole 14. Preferably only one through-hole 14 is formed at one fusion bond 4, and is formed at a specific position that has a predetermined positional relationship with the fusion bond 4. The through-hole 14 may have a shape in plan view that is similar to that of the fusion bond 4 at the outer periphery or may not have such a similar shape. Preferably the through-hole has a shape in plan view similar to that of the fusion bond at the outer periphery.

At the fusion bonds 4, resin having a property of thermal adhesiveness making up at least one of the first sheet 1 and the second sheet 2 melts and then is solidified, whereby the first sheet 1 and the second sheet 2 bond. When the first sheet 1 and the second sheet 2 are fiber sheets such as nonwoven fabric, fibers of the first sheet 1 and the second sheet 2 preferably melt or are embedded in the molten resin at the fusion bonds 4 so that the fibers cannot be observed visually, i.e., the sheets appear as films.

Next, the following describes a first embodiment of the method for manufacturing a composite sheet of the present invention.

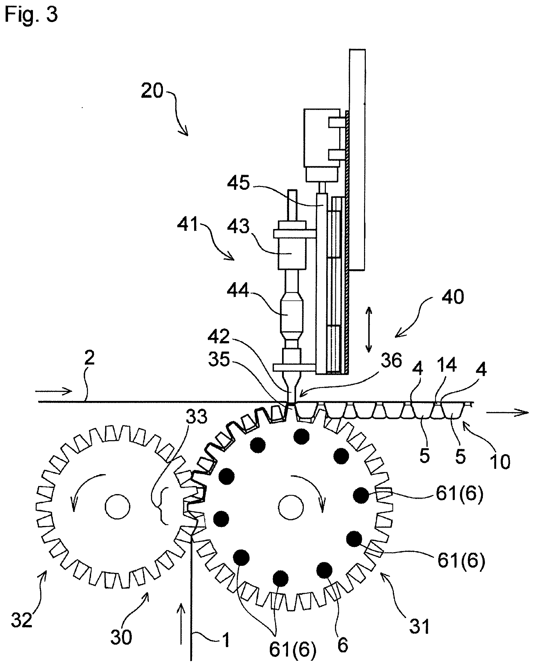

In the first embodiment, the composite sheet 10 as stated above is manufactured by an apparatus 20 for manufacturing a composite sheet that is the first embodiment shown in Fig. 3.

As shown in Fig. 3, the apparatus 20 for manufacturing a composite sheet includes: an embossing unit 30, an ultrasonic processing unit 40, and a preheater 6 to preheat a sheet before the application of ultrasonic vibrations to a predetermined controlled temperature.

As shown in Fig. 3, the embossing unit 30 includes first and second rollers 31 and 32 having projections and depressions engaging with each other on their peripheral surfaces. While rotating these rollers 31 and 32, the first sheet 1 is introduced to an engaging part 33 of these rollers 31 and 32 so as to deform the first sheet 1 to have depressions and projections following the depressions and projections of the first roller 31 on the peripheral surface.

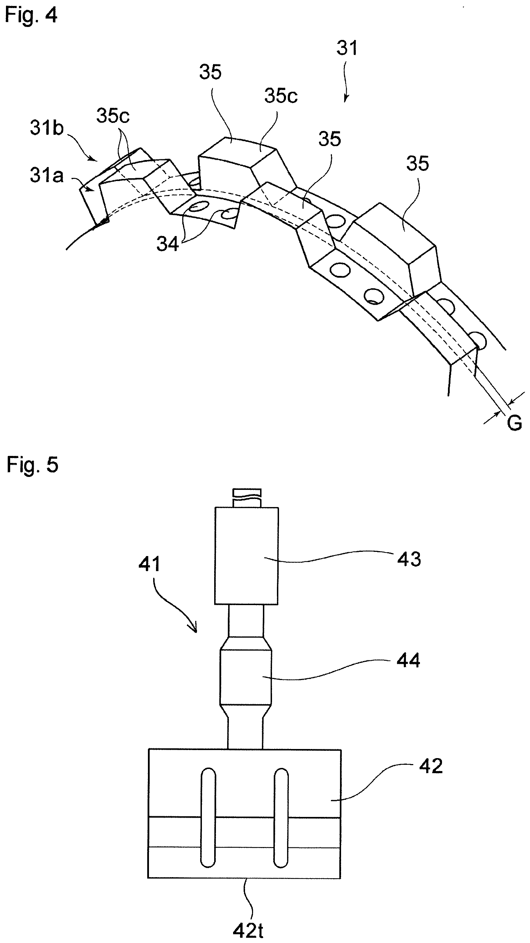

Fig. 4 is an enlarged perspective view of the major part of the first roller 31. Fig. 4 is a partial view of the peripheral surface of the first roller 31.

The first roller 31 is an assembly of a plurality of spur gears 31a, 31b, ... to have a roller form, and each gear has a given tooth width. Teeth of these gears form the projections 35 of the first roller 31 on the peripheral surface, and the top face 35c of each projection 35 forms a pressurizing face to pressurize the first and second sheets 1 and 2 as workpieces for bonding together with a distal-end face 42t of an ultrasonic horn 42 of an ultrasonic bonding machine 41 described later.

The tooth width (length of the gear in the axial direction) of each gear decides the dimension of the projections 5 of the composite sheet 10 in direction X, and the tooth thickness (length of the gear in the rotating direction) of each gear decides the dimension of the projections 5 of the composite sheet 10 in direction Y. The adjacent gears are assembled so that their teeth are out of alignment by half of the pitch. As a result, the first roller 31 has projections and depressions on its peripheral surface. In the present embodiment, the top face 35c of each projection 35 is a rectangle having a long side along the rotating direction of the first roller 31 and a short side along the axial direction of the first roller 31. Such a shape of the top face 35c that is longer in the rotating direction is preferable because it can lengthen the time when one of the projections 35 of the first roller 31 comes in contact with the distal-end face 42t of the ultrasonic horn 42 and so can raise the temperature easily.

The bottom land of each gear of the first roller 31 corresponds to the depressions on the peripheral surface of the first roller 31. The bottom land of each gear has suction holes 34 at the bottom. The suction holes 34 lead to a suction source (not illustrated), such as a blower or a vacuum pump, and they are controlled so that suction is performed from the engaging part 33 of the first roller 31 and the second roller 32 to the place where the first sheet 1 and the second sheet 2 join. With this configuration, the deformed first sheet 1 having depressions and projections due to the engagement between the first roller 31 and the second roller 32 keeps deformed having the shape following depressions and projections of the first roller 31 due to the suction force from the suction holes 34 until it reaches the place where the first sheet 1 and the second sheet 2 join and the application unit 36 of the ultrasonic bonding machine to apply ultrasonic vibrations.

As shown in Fig. 4, in this case, adjacent gears are preferably mounted with a predetermined gap G therebetween so that the first sheet 1 can be deformed following the shape on the peripheral surface of the first roller 31 without imposing excessive stretching force or a cutting effect of the engaging part 33 of these rollers 31 and 32 to the first sheet 1.

The second roller 32 has depressions and projections on its peripheral surface so as to engage with the depressions and projections on the peripheral surface of the first roller 31. The second roller 32 has a configuration similar to the first roller 31 other than that the second roller does not have suction holes 34. While rotating the first and second rollers 31 and 32 having mutually engaging depressions and projections, the first sheet 1 is introduced to the engaging part 33 of these rollers 31 and 32, whereby the first sheet 1 can be deformed to have depressions and projections. At the engaging part 33, the projections of the second roller 32 pushes the first sheet 1 at a plurality of parts into the depressions on the peripheral surface of the first roller 31, so that these pushed parts form the projections 5 of the resultant composite sheet 10. While the second roller 32 has a plurality of projections on the peripheral surface to be inserted into the depressions of the first roller 31, the second roller 32 does not have to have projections corresponding to all of the depressions on the first roller 31.

As shown in Fig. 3, the ultrasonic processing unit 40 includes the ultrasonic bonding machine 41 having the ultrasonic horn 42. After overlapping the second sheet 2 with the deformed first sheet 1 having depressions and projections, the ultrasonic processing unit pinches these sheets between the projections of the first roller 31 and the ultrasonic horn 42 and applies ultrasonic vibrations partially to form the fusion bond 4 having the through-hole 14.

As shown in Figs. 3 and 5, the ultrasonic bonding machine 41 includes an ultrasonic oscillator (not illustrated), a converter 43, a booster 44 and the ultrasonic horn 42. The ultrasonic oscillator (not illustrated) electrically connects to the converter 43 so that the converter 43 receives electrical signal of high voltage and of a wavelength at the frequency of about 15 kHz to 50 kHz that is generated by the ultrasonic oscillator. The ultrasonic oscillator (not illustrated) is mounted on a movable base 45 or outside of the movable base 45.

The converter 43 internally has an electrical device, such as a piezoelectric element, to convert an electrical signal input from the ultrasonic oscillator to mechanical vibrations. The booster 44 adjusts, preferably amplifies the amplitude of the mechanical vibrations issued from the converter 43 to transmit them to the ultrasonic horn 42. The ultrasonic horn 42 is made of a mass of metal, such as aluminum alloy or titanium alloy, and is designed to resonate correctly at the frequency for operation. Ultrasonic vibrations transmitted from the booster 44 to the ultrasonic horn 42 are amplified or attenuated inside of the ultrasonic horn 42 as well and are applied to the first and second sheets 1 and 2 as workpieces. Such an ultrasonic bonding machine 41 may be an assembly of commercially-available ultrasonic horn, converter, booster and ultrasonic oscillator.

The ultrasonic bonding machine 41 is fixed on the movable base 45. The clearance between the distal-end face 42t of the ultrasonic horn 42 and the top faces 35c of the projections 35 of the first roller 31 as well as the pressurizing force against the laminated first and second sheets 1 and 2 are therefore adjustable by moving the movable base 45 toward or away from the peripheral surface of the first roller 31.

While pressurizing the first and second sheets pinched between the top faces 35c of the projections 35 of the first roller 31 and the distal-end face 42t of the ultrasonic horn 42 of the ultrasonic bonding machine 41, ultrasonic vibrations are applied to the first and second sheets 1 and 2 as workpieces, whereby parts of the first sheet 1 located on the top faces 35c of the projections 35 fusion-bonds with the second sheet 2 to form fusion bonds 4 and through-holes 14 penetrating through both of the sheets 1 and 2 are formed to be surrounded with the molten parts.

In a preferable embodiment, the apparatus 20 for manufacturing a composite sheet includes the preheater 6 having a heater 61 disposed in the first roller 31. More specifically, the preheater includes a heating means, such as the heater 61, disposed in the first roller 31, a temperature-measuring means (not illustrated) that can measure the temperature of the sheet before application of ultrasonic vibrations, and a temperature controller (not illustrated) to control the temperature of the heater 61 in accordance with the measurement of the temperature-measuring means. The temperature to heat the peripheral surface of the first roller 31 by the heater 61 is controlled in accordance with the measurement of the temperature-measuring means, whereby the temperature of the first sheet 1 immediately before application of ultrasonic vibrations can be controlled precisely to a desired temperature.

In a preferable embodiment, the heater 61 is embedded in the first roller 31 along the axial direction. A plurality of the heaters 61 may be disposed around the rotary shaft of the first roller 31 and in the vicinity of the peripheral surface, and may be spaced away from each other in the circumferential direction. The temperature to heat the peripheral surface of the first roller 31 by the heater 61 is controlled by the temperature controller not illustrated so that the temperature of the first sheet 1 to be introduced into the application unit 36 to apply ultrasonic vibrations can be kept at temperatures within a predetermined range during the operation of the apparatus 20 for manufacturing a composite sheet.

Preferably the preheater 6 includes a heating means that applies thermal energy to a target from the outside to heat the target. Examples of the heating means include a cartridge heater having electrically heated wire, and any type of well-known heating means may be used without limitation.

The ultrasonic bonding machine applies ultrasonic vibrations to a workpiece to generate heat at the workpiece for melting and fusion-bonding, and the heating means in this description does not include the ultrasonic bonding machine.

As shown in Fig. 3, in the manufacturing method of the first embodiment of the present invention, the first sheet 1 fed from a stock roll (not illustrated) is introduced to the engaging part 33 of the first and second rollers 31 and 32 while these rollers 31 and 32 are rotating so as to deform the first sheet to have depressions and projections (embossing step). Then the deformed first sheet 1 having depressions and projections is conveyed while being held on the first roller 31, and the second sheet 2 fed from another stock roller (not illustrated) different from that of the first sheet 1 is overlapped with the first sheet 1 being conveyed (overlapping step). Then the overlapped sheets 1 and 2 are pinched between the projections 35 of the first roller 31 and the ultrasonic horn 42 of the ultrasonic bonding machine and ultrasonic vibrations is applied to the sheets (ultrasonic processing step). At the ultrasonic processing step, the fusion bonds 4 having through-holes 14 are formed during application of ultrasonic vibrations.

In the method for manufacturing of the first embodiment, prior to the application of ultrasonic vibrations, at least one of the first sheet 1 and the second sheet 2 is preferably heated at a temperature lower than the melting point of the sheet and not lower than the 50°C lower temperature of the melting point. That is, prior to application of ultrasonic vibrations, any one of the following (1) and (2) or both of them are preferably conducted:
(1) heating the first sheet 1 to a temperature lower than the melting point of the first sheet 1 and not lower than the 50°C lower temperature of the melting point; and
(2) heating the second sheet 2 to a temperature lower than the melting point of the second sheet 2 and not lower than the 50°C lower temperature of the melting point.

Preferably the first sheet 1 is heated to a temperature lower than the melting point of the first sheet 1 and not lower than the 50°C lower temperature of the melting point, and the second sheet 2 is heated to a temperature lower than the melting point of the second sheet 2 and not lower than the 50°C lower temperature of the melting point.

To control the temperature of the first sheet 1 at a temperature lower than the melting point of the first sheet 1 and not lower than the 50°C lower temperature of the melting point, the temperature of the first sheet 1 on the first roller 31 is measured between the engaging part 33 of the first and second rollers 31 and 32 and the application unit 36 of the ultrasonic bonding machine to apply ultrasonic vibrations, and controls the temperature on the peripheral surface of the first roller 31 so that the measurement is within the specified range as stated above, for example. To preheat the first sheet 1 to a temperature within a specific range, various methods may be used instead of controlling the temperature of the peripheral surface of the first roller 31 to be within the specified range of temperatures by the heater disposed in the first roller 31. For instance, a heater, an outlet of hot air or an irradiation device of far-infrared ray may be disposed in the vicinity of the peripheral surface of the first roller 31 to control the temperature of the first roller 31 on the peripheral surface before or after bringing the first sheet 1 in contact with the roller. The second roller 32 to come in contact with the first sheet 1 at the engaging part 33 may be heated, and the temperature of the first sheet 1 may be controlled by controlling the temperature of the peripheral surface of the second roller. A heated roller may be brought into contact with the first sheet 1 before coming in contact with the first roller 31, such a sheet may pass through a space kept at high temperatures, or hot air may be blown to such a sheet.

On the other hand, to control the temperature of the second sheet 2 at a temperature lower than the melting point of the second sheet 2 and higher than the 50°C lower temperature of the melting point, the method preferably measures the temperature of the second sheet before joining with the first sheet 1 by temperature-measuring means disposed along the conveyance path of the second sheet, and controls the temperature of the heating means (not illustrated) of the second sheet disposed along the conveyance path of the second path so that the measurement is within the specified range as stated above. The heating means of the second sheet may be of a contact type that brings a heated roller into contact with the sheet or of a non-contact type that passes the sheet through a space kept at high temperatures, blows hot air to the sheet or blows hot air so as to pass through the sheet, or irradiates the sheet with infrared ray.

The melting points of the first sheet 1 and the second sheet 2 may be measured by the following method.

For instance, the melting points may be measured with a differential scanning calorimeter (DSC) PYRIS Diamond DSC produced by Perkin-Elmer Co., Ltd. The melting points can be obtained from the peak values of the measurement data. When the first sheet 1 or the second sheet 2 is a fiber sheet, such as nonwoven fabric, and is a composite sheet including a plurality of components of the fibers as in a core-in-sheath or side-by-side configuration, the melting point of the sheet is the melting point at the lowest temperature among a plurality of melting points measured by the DSC.

In this way, in the method for manufacturing a composite sheet of the first embodiment, these overlapped first and second sheets 1 and 2 are pinched between the projections of the first roller 31 and the ultrasonic horn 42 of the ultrasonic bonding machine and applies ultrasonic vibrations to the sheets to form the fusion bonds 4 having the through-holes 14. In the method for manufacturing a composite sheet of the first embodiment, at least one of the first sheet 1 and the second sheet 2 is preferably preheated at a temperature in the specific range as stated above so that the sheet does not melt, and then ultrasonic vibrations are applied to both of the sheets 1 and 2, one of which or both of which has been preheated. At the step of applying ultrasonic vibrations, the condition to apply ultrasonic vibrations, such as the wavelength or the intensity of ultrasonic vibrations applied or the pressure applied to both of the sheets 1 and 2, are adjusted so that the ultrasonic vibrations melt both of the sheets 1 and 2 to form fusion bonds 4 and through-holes 14 penetrating through both of the sheets 1 and 2 are formed so as to be surrounded with the molten parts.

In this way, according to the method for manufacturing a composite sheet of the first embodiment, by preheating at least one of the first sheet 1 and the second sheet 2, preferably both of the sheets by a heating means, such as a heater, at a high temperature such that the sheet does not melt, and then pressurizing the sheets between the projections 35 of the first roller 31 and the ultrasonic horn 42 of the ultrasonic bonding machine while applying ultrasonic vibrations to form the fusion bonds 4 having the through-holes 14, the fusion bonds 4 having the through-holes 14 can be formed more correctly than the case without preheating of both of the sheets 1 and 2, and can suppress problems, such as adherence of molten resin to the conveyance means or the sheet being caught around the conveyance roller, which may occur when the both of the sheets 1 and 2 are preheated to a temperature exceeding the melting points. The burden for maintenance of the apparatus therefore can be small.

The fusion bonds 4 and the through-holes 14 can be formed at the same time between the projections 35 of the first roller 31 and the ultrasonic horn 42 of the ultrasonic bonding machine, and so the method can avoid displacement between the fusion bonds 4 and the through-holes 14.

The composite sheet 10 manufactured by the method of the first embodiment has depressions and projections and the fusion bond 4 having through-holes 14 at the bottom of the depressions, and so can give comfortable feel to the skin while in use and can prevent liquid from spreading along the surface of the sheet, and can have properties of allowing air to pass through easily and of wicking liquid easily.

The composite sheet 10 having such properties is favorably used for a topsheet of an absorbent article, and the use of the composite sheet 10 is not limited to such a topsheet.

To obtain one or more of the advantageous effects as stated above more reliably, the method and the apparatus for manufacturing a composite sheet of the present invention each preferably has the following configuration:
(1) preferably the first sheet 1 is preheated to a temperature not lower than the 50°C lower temperature of the melting point of the first sheet and lower than the melting point, and more preferably is preheated to a temperature not lower than the 20°C lower temperature of the melting point of the first sheet 1, and not higher than the 5°C lower temperature of the melting point.
(2) preferably the second sheet 2 is preheated to a temperature not lower than the 50°C lower temperature of the melting point of the second sheet 2 and lower than the melting point, and more preferably is preheated to a temperature not lower than the 20°C lower temperature of the melting point of the second sheet 2 and not higher than the 5°C lower temperature of the melting point.

For easy formation of the through-holes 14, the preheating temperature of the first sheet 1 and the second sheet 2 is preferably 100°C or higher and more preferably 130°C or higher. To prevent adherence of the sheet to the conveyance means or the sheet being caught around the conveyance roller, the preheating temperature is preferably 150°C or lower and more preferably 145°C or lower.

For easy formation of the fusion bonds 4 and the through-holes 14, the pressurizing force applied to the first and second sheets 1 and 2 pinched between the top faces 35c of the projections 35 of the first roller 31 and the distal-end face 42t of the ultrasonic horn 42 is preferably 10 N/mm or more and more preferably 15 N/mm or more, or is preferably 150 N/mm or less and more preferably 120 N/mm or less. The pressurizing force is preferably 10 N/mm or more and 150 N/mm or less and is more preferably 15 N/mm or more and 120 N/mm or less.

Herein, the pressurizing force means linear pressure, which indicates the value (pressure per unit length) obtained by dividing the pressurizing force (N) of the ultrasonic horn 42 by the total length (not including the depressions of the first roller 31) of the tooth width (direction X) of the projections 35 that come in contact with the ultrasonic horn 42.

For easy formation of the fusion bonds 4 and the through-holes 14, the frequency of ultrasonic vibrations applied is preferably 15 kHz or more and more preferably 20 kHz or more, or is preferably 50 kHz or less and more preferably 40 kHz or less. The frequency is preferably 15 kHz or more and 50 kHz or less and is more preferably 20 kHz or more and 40 kHz or less.

### [Method for measuring frequency]

Displacement of the horn at the distal end is measured by a laser displacement meter, for example. The frequency may be measured by setting the sampling rate at 200 kHz or more and the accuracy at 1 µm or more.

For easy formation of the fusion bonds and the through-holes, the amplitude of ultrasonic vibrations applied is preferably 20 µm or more and more preferably 25 µm or more, or is preferably 50 µm or less and more preferably 40 µm or less. The amplitude is preferably 20 µm or more and 50 µm or less and is more preferably 25 µm or more and 40 µm or less.

### [Method for measuring amplitude]

Displacement of the horn at the distal end is measured by a laser displacement meter, for example. The amplitude may be measured by setting the sampling rate at 200 kHz or more and the accuracy at 1 µm or more.

Next the following describes second and third embodiments of the method for manufacturing a composite sheet of the present invention.

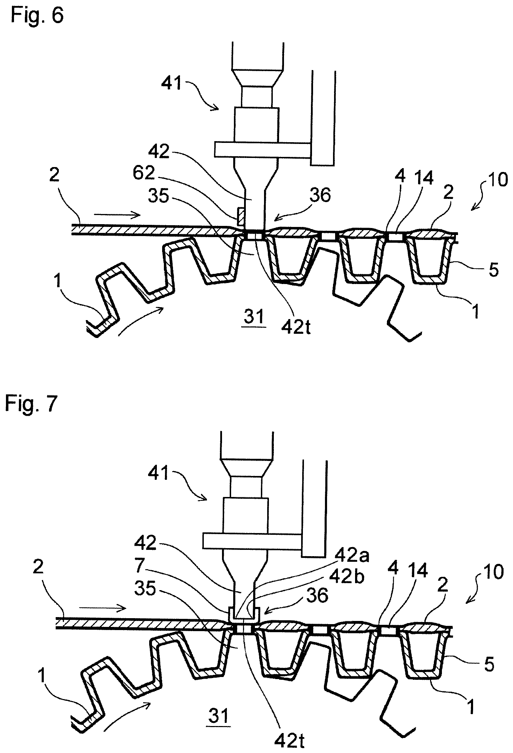

In the second embodiment, the composite sheet 10 as stated above is manufactured by an apparatus for manufacturing a composite sheet that is the second embodiment having a major part shown in Fig. 6.

The apparatus for manufacturing a composite sheet of the second embodiment is different from the apparatus 20 for manufacturing a composite sheet as stated above only in that it includes a means to heat the ultrasonic horn 42 of the ultrasonic bonding machine 41 instead of the heater 61 (preheater) disposed in the first roller 31. More specifically, the apparatus for manufacturing a composite sheet of the second embodiment includes a heater 62 mounted to the ultrasonic horn 42 that is the means to heat the ultrasonic horn 42.

The method for manufacturing a composite sheet of the second embodiment controls the temperature of the ultrasonic horn 42 that is heated by the heater 62 so that the temperature of the second sheet 2 immediately before application of ultrasonic vibrations is heated at a temperature less than the melting point of the sheet 2 and not lower than the 50°C lower temperature of the melting point, and applies ultrasonic vibrations to the first and second sheets 1 and 2 pinched between the projections 35 of the first roller 31 and the ultrasonic horn 42 of the ultrasonic bonding machine while keeping the temperature.

In such a configuration of heating the ultrasonic horn 42 by the heating means, such as the heater 62, it is difficult to measure the temperatures of the sheets heated by the preheater because the first and second sheets 1 and 2 receiving ultrasonic vibrations from the ultrasonic bonding machine generate heat. To cope with this, when one of or both of the first and second sheets 1 and 2 is preheated by the heated ultrasonic horn 42, only heating is performed for 30 minutes without generating ultrasonic vibrations. Subsequently, the temperature of the distal-end face 42t of the ultrasonic horn is measured, and the measurement is used for the temperature of the sheet(s) heated by the preheater.

The second embodiment is similar to the first embodiment unless otherwise stated, and so the description for the first embodiment is applicable as needed.

In the third embodiment, the composite sheet 10 as stated above is manufactured by an apparatus for manufacturing a composite sheet that is the third embodiment having a major part shown in Fig. 7.

The apparatus for manufacturing a composite sheet of the third embodiment does not include a preheater to, prior to application of ultrasonic vibrations, heat at least one of the first sheet and the second sheet to a predetermined temperature. Instead, the apparatus includes a member 7 having a heat-storage property (hereinafter this may be called a "heat-storage member") at the distal-end part of the ultrasonic horn 42.

Similarly to the manufacturing method of the first embodiment, in the method for manufacturing a composite sheet of the third embodiment, the first sheet 1 is introduced to the engaging part 33 of the first and second rollers 31 and 32 to deform the first sheet to have depressions and projections. Then the deformed first sheet 1 having depressions and projections is conveyed toward the application unit 36 to apply ultrasonic vibrations while holding the first sheet 1 on the first roller 31. The second sheet 2 is overlapped with the first sheet 1 being conveyed, and then these overlapped first and second sheets 1 and 2 are pinched between the projections 35 of the first roller 31 and the distal-end face 42t of the ultrasonic horn 42 at the application unit 36 of ultrasonic vibrations and applies ultrasonic vibrations to the sheets.

As shown in Fig. 7, in the method for manufacturing of the third embodiment, these ultrasonic vibrations are applied while the heat-storage member 7 is disposed at the distal-end part of the ultrasonic horn 42.

In the manufacturing method of the third embodiment, the temperature of the distal-end part of the ultrasonic horn 42 having the heat-storage member 7 is not higher than the melting points of the first and second sheets 1 and 2 immediately after a start of the operation of the apparatus for manufacturing a composite sheet. As the operation continues, the heat-storage member 7 stores heat of the first and second sheets 1 and 2 generated due to ultrasonic vibrations, and so the temperature of the heat-storage member 7 increases to be the melting points or more of the first and second sheets 1 and 2. Once the temperature of the heat-storage member 7 is the melting points or more of the first sheet 1 and the second sheet 2, then the method may adjust the condition to apply ultrasonic vibrations, such as the wavelength or the intensity of ultrasonic vibrations applied or the pressure applied to both of the sheets 1 and 2, so that the ultrasonic vibrations applied melt both of the sheets 1 and 2 and through-holes 14 penetrating through both of the sheets 1 and 2 are formed to be surrounded with the molten parts. This can form the fusion bonds 4 having the through-holes 14 accurately, and can prevent problems, such as adherence of molten resin that is the molten sheet to the conveyance means or the sheet being caught around the conveyance roller. The burden for maintenance of the apparatus therefore can be small.

The fusion bonds 4 and the through-holes 14 can be formed at the same time between the projections 35 of the first roller 31 and the ultrasonic horn 42 of the ultrasonic bonding machine, and so the method can avoid displacement between the fusion bonds 4 and the through-holes 14.

The heat-storage member 7 has a heat conductivity which is at least lower than that of the metal of the ultrasonic horn 42.

Preferably the heat-storage member 7 has a heat conductivity measured by the following method that is 2.0 W/mK or less. For less heat-dissipation to the ultrasonic horn or to the air, heat conductivity of the heat-storage member is preferably 2.0 W/mK or less, and more preferably 1.0 W/mK or less. To heat the sheet efficiently, heat conductivity is preferably 0.1 W/mK or more and more preferably 0.5 W/mK or more. Preferably heat conductivity is 0.1 W/mK or more and 2.0 W/mK or less, and more preferably 0.5 W/mK or more and 1.0 W/mK or less.

### [Method for measuring heat conductivity]

The heat conductivity of the heat-storage member 7 is measured by a heat-conductivity measurement device.

The heat-storage member 7 that has heat resistance is preferably used. Heatproof temperature of the heat-storage member 7 is preferably 150°C or more, more preferably 200°C or more, and even more preferably 250°C or more. The upper limit of the heatproof temperature is not specified, and the heatproof temperature is 1500°C or less, for example.

The heat-storage member 7 may include a body having a heat-storage property and an adhesive layer to bond the body to the ultrasonic horn. In that case, heat conductivity of the heat-storage member is measured for a part without the part of the adhesive. For such a heat-storage member, commercially available products, such as "glass-cloth adhesive tape" produced by Nitto Denko Corporation and "heat-resistant and insulating polyimide adhesive tape" produced by Nitto Denko Corporation, may be used.

The body of the heat-storage member 7 having a heat-storage property may be made of materials, such as glass and polyimide. Among them, polyimide is preferable because it storages heat moderately and is highly resistant to heat. The heat-storage member 7 may be a sheet having a single-layer structure or a laminate of two or more sheets made of the same material or different materials.

Preferably the heat-storage member 7 has an oblong shape that is longer in the axial direction of the first roller 31 similarly to the distal-end face of the ultrasonic horn 42 that is also long in such a direction. To prevent the sheet from being caught during application of ultrasonic vibrations, the heat-storage member 7 preferably has a part covering a corner 42a of the distal-end face 42t of the ultrasonic horn 42 located upstream (left in Fig. 7) in the sheet-flowing direction in addition to the part covering the distal-end face of the ultrasonic horn 42, and preferably has a part covering a corner 42b of the distal-end face 42t of the ultrasonic horn 42 located downstream (right in Fig. 7) in the sheet-flowing direction as shown in Fig. 7.

Polyimide used for the body of the heat-storage member 7 is preferable also because it is highly resistant to abrasion and heat, and is a synthetic resin having a property of generating heat by itself due to ultrasonic vibrations applied.

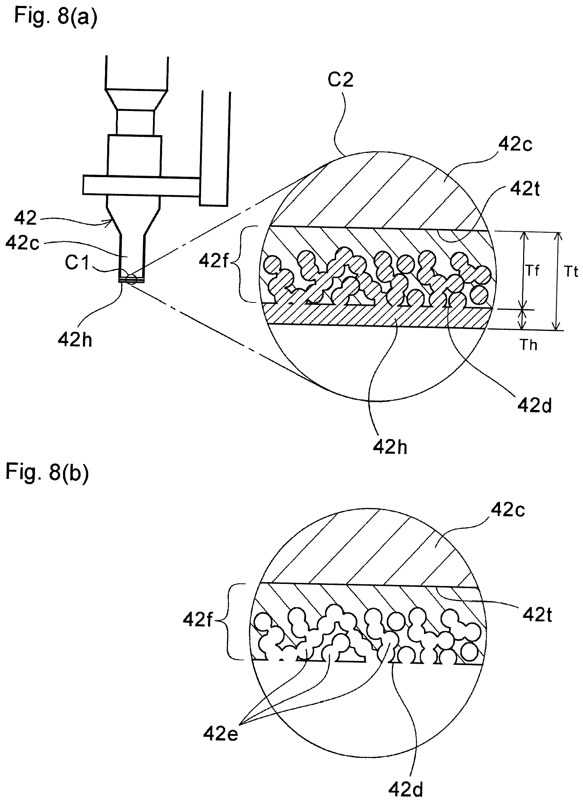

For similar viewpoints, the body of the heat-storage member 7 and the synthetic resin layer 42h of the ultrasonic horn 42 shown in Fig. 8(a) are preferably made of synthetic resins, such as polyimide, polybenzimidazole, polyetherethylketone, polyphenylenesulfide, polyetherimide, and polyamide-imide, that have Rockwell hardness of R120 or more and R140 or less and heatproof temperature of 150°C or more and 500°C or less. More preferably they may be made of synthetic resin, such as polyimide and polybenzimidazole, that have Rockwell hardness of R125 or more and R140 or less and heatproof temperature of 280°C or more and 400°C or less.

Herein, Rockwell hardness is a value measured in compliance with ASTM D785, and heatproof temperature is a value measured in compliance with ASTM D648.

The synthetic resin layer at the distal-end part of the ultrasonic horn 42 has a heatproof temperature that is preferably 150°C or more and more preferably 280°C or more, or is preferably 500°C or less and more preferably 400°C or less. Preferably the heatproof temperature is 150°C or more and 500°C or less, and more preferably 280°C or more and 400°C or less.

The synthetic resin layer at the distal-end part of the ultrasonic horn 42 has Rockwell hardness that is preferably R120 or more and more preferably R125 or more, or is preferably R140 or less. Preferably the Rockwell hardness is R120 or more and R140 or less, and is more preferably R125 or more and R140 or less.

Fig. 8(a) shows another example of the ultrasonic horn 42 having the heat-storage member 7 at the distal-end part. In Fig. 8(a), the circle C2 shows an enlarged cross-sectional view of the circle C1.

As shown in Fig. 8(b), the ultrasonic horn 42 of Fig. 8(a) includes a connecting layer 42f having voids 42e extending from one face 42d of the distal-end face 42t to the inside that are formed by thermal-spraying on the distal-end face 42t of the body 42c of the ultrasonic horn 42 made of metal, such as aluminum alloy or titanium alloy. The ultrasonic horn further includes a synthetic resin layer 42h having a heat-storage property that is fixed to the side of the one face 42d of the connecting layer 42f as shown in Fig. 8(a). Thermal spraying is a surface-treatment method to heat particles of a thermal-sprayed material, such as metals and ceramics, for melting or substantially melting, and accelerate the particles to let them collide with the surface of a substrate at high speed so as to form a coating on the surface of the substrate. While synthetic resin such as polyimide as the material of the synthetic resin layer 42h having a heat-storage property can be highly resistant to abrasion and heat, a sufficient fixing strength may not be achieved when such resin is directly fixed to the body. The ultrasonic horn of the present embodiment includes the synthetic resin layer 42h made of synthetic resin, such as polyimide, having a heat-storage property at the distal-end face 42t of the body 42c of the ultrasonic horn 42 made of metal such as titanium alloy via the connecting layer 42f formed by thermal-spraying, and so can have a sufficient strength for fixing easily. Note here that when the fixing strength is not sufficient, a problem, such as peel-off of the synthetic resin layer 42h, may be caused during the manufacturing of the composite sheet 10.

The connecting layer 42f may be made of any material as long as it can be thermal-sprayed, and can contribute to improvement of the fixing strength of the synthetic resin layer 42h having a heat-storage property. Ceramics, such as tungsten carbide, zirconia and chromium carbide, alloys such as aluminum-magnesium and zinc-aluminum, metals such as aluminum, stainless steel, titanium and molybdenum, and cermet that is a composite material of metal and ceramic are preferably used because these materials exert excellent binding with the body 42c of the ultrasonic horn 42 made of metal, such as titanium alloys and can be highly resistant to abrasion and heat. Among them, ceramics is preferable and tungsten carbide is more preferable because they can form voids 42e to increase the strength of the synthetic resin layer 42h for fixing.

Preferably, to increase the fixing strength of the synthetic resin layer 42h, the connecting layer 42f is made of a material having a melting point higher than that of the synthetic resin of the synthetic resin layer 42h having a heat-storage property and so keeps the shape of the voids 42e during formation of the synthetic resin 42h.

Examples of the method for fixing the synthetic resin layer 42h having a heat-storage property to the connecting layer 42f include immersing the connecting layer 42f in synthetic resin that is molten by heating, applying the synthetic resin that is molten by heating to the connecting layer 42f, and pressing a plate-like softened synthetic resin against the connecting layer 42f.

The thickness Tf (see Fig. 8(a)) of the connecting layer 42f is not limited especially. For example, the thickness is preferably 10 µm or more and more preferably 20 µm or more, or is preferably 100 µm or less and more preferably 50 µm or less. The thickness is preferably 10 µm or more and 100 µm or less, and more preferably 20 µm or more and 50 µm or less.

The thickness Th (see Fig. 8(a)) of the synthetic resin layer 42h having a heat-storage property is not limited especially. For example, the thickness is preferably 5 µm or more and more preferably 10 µm or more, or is preferably 100 µm or less and more preferably 50 µm or less. The thickness is preferably 5 µm or more and 100 µm or less, and more preferably 10 µm or more and 50 µm or less.

For the thickness Tf of the connecting layer 42f, the ratio of the thickness Tf to the total thickness Tt of the thickness Tf and the thickness Th of the synthetic resin layer 42h is preferably 30% or more and more preferably 50% or more, or is preferably 85% or less and more preferably 75% or less to keep the fixing strength of the synthetic resin and so as not to interfere with ultrasonic vibrations and heat generation. The ratio is preferably 30% or more and 85% or less, and more preferably 50% or more and 75% or less.

Such an apparatus for manufacturing a composite sheet including the heat-storage member 7 as in the third embodiment may include the preheater 6 of the apparatus for manufacturing a composite sheet in the first embodiment or the means to heat the ultrasonic horn 42 of the apparatus for manufacturing a composite sheet in the second embodiment.

Preferably the composite sheet 10 manufactured in these embodiments has the following structure.

Thickness H (see Fig. 1) of the projections 5 is 1 to 10 mm, and is preferably 3 to 6 mm. The number of the projections 5 per unit area (1 cm²) of the composite sheet 10 is 1 to 20, and is preferably 6 to 15. Dimension A of the bottom of the projection 5 in direction X (see Fig. 1) is 0.5 to 5.0 mm, and is preferably 1.0 to 4.0 mm. Dimension B of the bottom of the projection 5 in direction Y (see Fig. 1) is 1.0 to 10 mm, and is preferably 2.0 to 7.0 mm.

The ratio of bottom dimension A in direction X to bottom dimension B in direction Y (bottom dimension A : bottom dimension B) is 1:1 to 1:10, and is preferably 1:2 to 2:5. Area of the bottom of the projection 5 (bottom dimension A × bottom dimension B) is 0.5 to 50 mm², and is preferably 2 to 20 mm².

Preferably each fusion bond 4 has dimension C in direction X (see Fig. 1) that is 0.5 to 2 mm, and is preferably 0.8 to 1.5 mm, and dimension D in direction Y (see Fig. 1) that is 1.0 to 5.0 mm, and is preferably 1.2 to 3.0 mm. The ratio of dimension C in direction X to dimension D in direction Y (dimension C : dimension D) is 1:1 to 1:3, and is preferably 2:3 to 2:5.

Each fusion bond 4 has the area inside of the outer periphery that is preferably 0.5 mm² or more and more preferably 1.0 mm² or more, or is preferably 5.0 mm² or less and more preferably 4.0 mm² or less. Preferably the area is 0.5 mm² or more and 5.0 mm² or less, and more preferably 1.0 mm² or more and 4.0 mm² or less. The area of the fusion bond 4 inside of the outer periphery includes the area of the corresponding through-hole 14 as well.

The ratio of the opening area of the through-hole 14 to the area of the corresponding fusion bond 4 inside of the outer periphery is preferably 50% or more and more preferably 80% or more, or is preferably less than 100% and more preferably 95% or less. Preferably the ratio is 50% or more and less than 100%, and more preferably 80% or more and 95% or less.

The composite sheet 10 as stated above is favorably used as a topsheet of absorbent articles, such as disposable diapers, sanitary napkins, panty liners and incontinence pads.

The composite sheet may be used for purposes other than the topsheet of absorbent articles.

The composite sheet is also useful as another sheet for absorbent articles, and can be used, for example, as a sheet disposed between the topsheet and the absorbent member or a sheet for forming standing gathers (leak-proof cuffs), particularly a sheet forming the inner side of the gathers. It also finds use in applications other than absorbent articles, for example, as a cleaning sheet, particularly mainly to absorb liquid, or a cosmetic sheet for human body. In applications as a cleaning sheet, the projections are well conformable to a surface to be cleaned that is not smooth. The sheet is therefore preferably used with its first nonwoven fabric side in contact with a surface to be cleaned. In applications as a cosmetic sheet, the projections are well conformable to a skin of a target person and exert a massage effect, and are capable of absorbing excessively applied cosmetics (separately applied) or sweat, and so the sheet is preferably used with its first nonwoven fabric side in contact with the skin.

It should be understood that the method and apparatus for manufacturing a composite sheet of the present invention are not limited to the above embodiments, and various changes and modifications can be made as needed.

For instance, the composite sheet 10 as stated above has one fusion bond at each of the depressions. However, the composite sheet of the present invention may include a plurality of fusion bonds at one depression. The projection 5 of the composite sheet 10 as stated above is truncated four-sided pyramidal, and this may be hemispherical. The displacement of the projections 5 and the fusion bonds 4 in direction X with respect to those in the adjacent lines does not need to be a half the pitch, and may be, for example 1/3 or 1/4 the pitch. The projections 5 and fusion bonds 4 of adjacent lines may not be displaced in direction X.

The fusion bonds and the through-holes may have an oval or round shape or a polygonal shape (e.g., square, rectangle, triangle and diamond) having round corners in plan view.

The composite sheet 10 manufactured may have projections and fusion bonds in the form shown in Fig. 4 or 5 of JP 2016-116582 A or may have projections and fusion bonds in the form shown in Fig. 3 of JP 2016-116583 A. Instead of the structure having all of the fusion bonds of the composite sheet having their corresponding through-holes, a part of the fusion bonds of the composite sheet may have the through-holes. For instance, one of or both of the first and second sheets is preheated at the center region in the width direction of the continuous composite sheet to form fusion bonds having through-holes there. On the contrary, preheating is not performed to regions lateral to and sandwiching the center region of both of the first and second sheets, so as to form fusion bonds without through-hole there.

### Industrial Applicability

The method for manufacturing a composite sheet of the present invention can easily form fusion bonds having through-holes, and hardly causes displacement of the fusion bonds from the through-holes and can reduce the burden for maintenance of the apparatus.

The apparatus for manufacturing a composite sheet of the present invention can easily form fusion bonds having through-holes, and hardly causes displacement of the fusion bonds from the through-holes and can reduce the burden for maintenance.

## Claims

1. A method for manufacturing a composite sheet including a large number of fusion bonded parts where a first sheet and a second sheet are fusion-bonded, the first sheet having projections projecting to the opposite side of the second sheet at least partially at parts other than the fusion bonds, the method comprising:
an embossing step of introducing the first sheet to an engaging part of a first roller and a second roller each having projections and depressions engaging with each other on peripheral surfaces while rotating both of the first and second rollers to deform the first sheet to have depressions and projections;
an overlapping step of conveying the deformed first sheet having depressions and projections while holding the first sheet on the first roller, and overlapping the second sheet with the first sheet being conveyed; and
an ultrasonic processing step of pinching the overlapped first and second sheets between the projections of the first roller and an ultrasonic horn of an ultrasonic bonding machine to apply ultrasonic vibrations to the sheets,
wherein at the ultrasonic processing step, the fusion bonds having through-holes are formed during application of the ultrasonic vibrations.

2. The method for manufacturing a composite sheet according to claim 1, wherein the ultrasonic horn is heated to a predetermined temperature, and at the ultrasonic processing step, at least one of the first sheet and the second sheet is heated while applying the ultrasonic vibrations.

3. The method for manufacturing a composite sheet according to claim 1 or 2, wherein the first roller is heated to a predetermined temperature, and prior to application of the ultrasonic vibrations, at least one of the first sheet and the second sheet is heated.

4. The method for manufacturing a composite sheet according to any one of claims 1 to 3, wherein prior to application of the ultrasonic vibrations, at least one of the first sheet and the second sheet is directly heated to a predetermined temperature.

5. The method for manufacturing a composite sheet according to any one of claims 2 to 4, wherein the predetermined temperature is lower than a melting point of the first sheet or the second sheet to be heated and higher than a 50°C lower temperature of the melting points.

6. The method for manufacturing a composite sheet according to any one of claims 1 to 5, wherein at the ultrasonic processing step, the ultrasonic vibrations is applied while a member having a heat-storage property is disposed at a distal-end part of the ultrasonic horn so as to form the fusion bonds having through-holes during application of the ultrasonic vibrations.

7. The method for manufacturing a composite sheet according to claim 6, wherein the member having a heat-storage property has a heat conductivity lower than a heat conductivity of a material of the ultrasonic horn.

8. An apparatus for manufacturing a composite sheet including a large number of fusion bond parts where a first sheet and a second sheet are fusion-bonded, the first sheet having projections projecting to the opposite side of the second sheet at least partially at parts other than the fusion bonds, the apparatus comprising:
an embossing unit including a first roller and a second roller each having projections and depressions engaging with each other on peripheral surfaces, the embossing unit being configured to deform the first sheet introduced to an engagement part of the first and second rollers to have depressions and projections; and
an ultrasonic processing unit including an ultrasonic bonding machine and being configured to overlap the second sheet with the deformed first sheet having depressions and projections and pinch the overlapped first and second sheets between the projections of the first roller and an ultrasonic horn of the ultrasonic bonding machine to partially apply ultrasonic vibrations to the sheets and form the fusion bonds having through-holes.

9. The apparatus for manufacturing a composite sheet according to claim 8, wherein the apparatus further comprises a means to heat the ultrasonic horn.

10. The apparatus for manufacturing a composite sheet according to claim 8 or 9, wherein the apparatus further comprises a preheater to preheat at least one of the first sheet and the second sheet to a predetermined temperature prior to application of the ultrasonic vibrations.

11. The apparatus for manufacturing a composite sheet according to claim 10, wherein the preheater is a heater disposed in the first roller.

12. The apparatus for manufacturing a composite sheet according to any one of claims 8 to 11, wherein a member having a heat-storage property is disposed at a distal-end part of the ultrasonic horn.

13. The apparatus for manufacturing a composite sheet according to claim 12, wherein the member having a heat-storage property has a heat conductivity lower than a heat conductivity of a material of the ultrasonic horn.

14. The apparatus for manufacturing a composite sheet according to any one of claims 12 to 13, wherein the member having a heat-storage property includes glass or polyimide.

15. The apparatus for manufacturing a composite sheet according to any one of claims 12 to 13 wherein the ultrasonic horn includes, at the distal-end part, a synthetic resin layer that is resistant to heat as a member having a heat-storage property.

## Patentansprüche

1. Verfahren zum Herstellen einer Verbundfolie, die eine hohe Anzahl von schmelzverbundenen Teilen aufweist, wobei eine erste Folie und eine zweite Folie schmelzverbunden werden, wobei die erste Folie Vorsprünge hat, die zur gegenüberliegenden Seite der zweiten Folie wenigstens teilweise an Teilen, die sich von den Schmelzverbindungen unterscheiden, hervorstehen, wobei das Verfahren aufweist:
einen Prägeschritt des Einsetzens der ersten Folie in ein Eingriffsteil einer ersten Rolle und einer zweiten Rolle, von denen jede Vorsprünge und Vertiefungen hat, die miteinander an Umfangsflächen eingreifen, während beide von der ersten und der zweiten Rolle rotiert werden, um die erste Folie zu verformen, um Vertiefungen und Vorsprünge zu haben;
einen Überlappungsschritt des Transportieren der verformten ersten Folie, die Vertiefungen und Vorsprünge hat, während die erste Folie auf der ersten Rolle gehalten wird, und des Überlappens der zweiten Folie mit der transportierten ersten Folie; und
einen Ultraschallverarbeitungsschritt des Einklemmens der überlappten ersten und zweiten Folie zwischen den Vorsprüngen der ersten Rolle und einen Ultraschalltrichter einer Ultraschallschweißmaschine, um Ultraschallvibrationen auf die Folien anzulegen,
wobei im Ultraschallverarbeitungsschritt während des Anlegens der Ultraschallvibrationen die Schmelzverbindungen mit Durchgangsöffnungen ausgebildet werden.

2. Verfahren zum Herstellen einer Verbundfolie nach Anspruch 1, wobei der Ultraschalltrichter auf eine vorbestimmte Temperatur erwärmt wird, und im Ultraschallverarbeitungsschritt wenigstens eine von der ersten Folie und der zweiten Folie erhitzt wird, während Ultraschallvibrationen angelegt werden.

3. Verfahren zum Herstellen einer Verbundfolie nach Anspruch 1 oder 2, wobei die erste Rolle auf eine vorbestimmte Temperatur erwärmt wird, und vor dem Anlegen der Ultraschallvibrationen wenigstens eine von der ersten Folie und der zweiten Folie erhitzt wird.

4. Verfahren zum Herstellen einer Verbundfolie nach einem der Ansprüche 1 bis 3, wobei vor dem Anlegen der Ultraschallvibrationen wenigstens eine von der ersten Folie und der zweiten Folie direkt auf eine vorbestimmte Temperatur erhitzt wird.

5. Verfahren zum Herstellen einer Verbundfolie nach einem der Ansprüche 2 bis 4, wobei die vorbestimmte Temperatur niedriger als ein Schmelzpunkt der zu erwärmenden ersten Folie oder der zweiten Folie ist und höher als eine 50 °C niedrigere Temperatur der Schmelzpunkte.

6. Verfahren zum Herstellen einer Verbundfolie nach einem der Ansprüche 1 bis 5, wobei im Ultraschallverarbeitungsschritt die Ultraschallvibrationen angelegt werden, während ein Element mit einer Wärmespeichereigenschaft an einem distalen Endteil des Ultraschalltrichters angeordnet ist, um so die Schmelzverbindungen mit Durchgangsöffnungen während des Anlegens der Ultraschallvibrationen zu bilden.

7. Verfahren zum Herstellen einer Verbundfolie nach Anspruch 6, wobei das Element mit Wärmespeichereigenschaft eine Wärmeleitfähigkeit hat, die niedriger als eine Wärmeleitfähigkeit eines Materials des Ultraschalltrichters ist.

8. Vorrichtung zum Herstellen einer Verbundfolie, die eine hohe Anzahl von schmelzverbundenen Teilen aufweist, wobei eine erste Folie und eine zweite Folie schmelzverbunden sind, die erste Folie Vorsprünge hat, die zur gegenüberliegenden Seite der zweiten Folie wenigstens teilweise an Teilen, die sich von den Schmelzverbindungen unterscheiden, hervorstehen, wobei die Vorrichtung aufweist:
eine Prägeeinheit, die eine erste Rolle und eine zweite Rolle aufweist, von denen jede Vorsprünge und Vertiefungen hat, die miteinander an Umfangsflächen eingreifen, wobei die Prägeeinheit eingerichtet ist, die erste Folie zu verformen, die an einem Eingriffsteil der ersten und zweiten Rolle eingesetzt ist, um Vertiefungen und Vorsprünge zu haben; und
eine Ultraschallverarbeitungseinheit, die eine Ultraschallschweißmaschine aufweist und eingerichtet ist, die zweite Folie mit der verformten ersten Folie mit Vertiefungen und Vorsprüngen zu überlappen, und die überlappte erste und zweite Folie zwischen den Vorsprüngen der ersten Rolle und einem Ultraschalltrichter einer Ultraschallschweißmaschine einzuklemmen, um teilweise Ultraschallvibrationen auf die Folien anzulegen und die Schmelzverbindungen mit Durchgangsöffnungen auszubilden.

9. Vorrichtung zum Herstellen einer Verbundfolie nach Anspruch 8, wobei die Vorrichtung weiterhin ein Mittel aufweist, um den Ultraschalltrichter zu erwärmen.

10. Vorrichtung zum Herstellen einer Verbundfolie nach Anspruch 8 oder 9, wobei die Vorrichtung weiterhin eine Vorwärmeeinrichtung aufweist, um wenigstens eine von der ersten Folie und der zweiten Folie vor Anlegen der Ultraschallvibrationen auf eine bestimmte Temperatur vorab zu erwärmen.

11. Vorrichtung zum Herstellen einer Verbundfolie nach Anspruch 10, wobei die Vorwärmeeinrichtung eine Heizung ist, die in der ersten Rolle angeordnet ist.

12. Vorrichtung zum Herstellen einer Verbundfolie nach einem der Ansprüche 8 bis 11, wobei ein Element mit Wärmespeichereigenschaft an einem distalen Endteil des Ultraschalltrichters angeordnet ist.

13. Vorrichtung zum Herstellen einer Verbundfolie nach Anspruch 12, wobei das Element mit Wärmespeichereigenschaft eine Wärmeleitfähigkeit hat, die niedriger als die Wärmeleitfähigkeit eines Materials des Ultraschalltrichters ist.

14. Vorrichtung zum Herstellen einer Verbundfolie nach einem der Ansprüche 12 bis 13, wobei ein Element mit Wärmespeichereigenschaft Glas oder Polyimid aufweist.

15. Vorrichtung zum Herstellen einer Verbundfolie nach einem der Ansprüche 12 bis 13, wobei der Ultraschalltrichter an dem distalen Endteil eine hitzebeständige Kunstharzschicht als ein Element mit Wärmespeichereigenschaft aufweist.

## Revendications

1. Méthode pour fabriquer une feuille composite comportant un grand nombre de parties soudées par fusion où une première feuille et une seconde feuille sont soudées par fusion, la première feuille ayant des saillies faisant saillie vers le côté opposé de la seconde feuille au moins partiellement au niveau de parties autres que les soudures par fusion, la méthode comprenant :
une étape de gaufrage consistant à introduire la première feuille dans une partie de mise en prise d'un premier rouleau et d'un second rouleau ayant chacun des saillies et des creux venant en prise les uns avec les autres sur des surfaces périphériques tout en faisant tourner à la fois les premier et second rouleaux pour déformer la première feuille pour avoir des creux et des saillies ;
une étape de recouvrement consistant à transporter la première feuille déformée ayant des creux et des saillies tout en maintenant la première feuille sur le premier rouleau, et recouvrir la seconde feuille avec la première feuille en train d'être transportée ; et
une étape de traitement par ultrasons consistant à pincer les première et seconde feuilles recouvertes entre les saillies du premier rouleau et un émetteur d'ultrasons d'une machine de soudage par ultrasons pour appliquer des vibrations ultrasonores aux feuilles,
dans laquelle à l'étape de traitement par ultrasons, les soudures par fusion ayant des trous traversants sont formées durant une application des vibrations ultrasonores.

2. Méthode pour fabriquer une feuille composite selon la revendication 1, dans laquelle l'émetteur d'ultrasons est chauffé jusqu'à une température prédéterminée, et à l'étape de traitement par ultrasons, au moins une parmi la première feuille et la seconde feuille est chauffée pendant l'application des vibrations ultrasonores.

3. Méthode pour fabriquer une feuille composite selon la revendication 1 ou 2, dans laquelle le premier rouleau est chauffé jusqu'à une température prédéterminée, et avant une application des vibrations ultrasonores, au moins une parmi la première feuille et la seconde feuille est chauffée.

4. Méthode pour fabriquer une feuille composite selon l'une quelconque des revendications 1 à 3, dans laquelle avant une application des vibrations ultrasonores, au moins une parmi la première feuille et la seconde feuille est directement chauffée jusqu'à une température prédéterminée.

5. Méthode pour fabriquer une feuille composite selon l'une quelconque des revendications 2 à 4, dans laquelle la température prédéterminée est inférieure à un point de fusion de la première feuille ou la seconde feuille devant être chauffée et supérieure à une température plus basse de 50 °C des points de fusion.

6. Méthode pour fabriquer une feuille composite selon l'une quelconque des revendications 1 à 5, dans laquelle à l'étape de traitement par ultrasons, les vibrations ultrasonores sont appliquées tandis qu'un élément ayant une propriété de stockage de chaleur est disposé au niveau d'une partie d'extrémité distale de l'émetteur d'ultrasons de manière à former les soudures par fusion ayant des trous traversants durant une application des vibrations ultrasonores.

7. Méthode pour fabriquer une feuille composite selon la revendication 6, dans laquelle l'élément ayant une propriété de stockage de chaleur a une conductivité thermique inférieure à une conductivité thermique d'un matériau de l'émetteur d'ultrasons.

8. Appareil pour fabriquer une feuille composite comportant un grand nombre de parties de soudure par fusion où une première feuille et une seconde feuille sont soudées par fusion, la première feuille ayant des saillies faisant saillie vers le côté opposé de la seconde feuille au moins partiellement au niveau de parties autres que les soudures par fusion, l'appareil comprenant :
une unité de gaufrage comportant un premier rouleau et un second rouleau ayant chacun des saillies et des creux venant en prise les uns avec les autres sur des surfaces périphériques, l'unité de gaufrage étant configurée pour déformer la première feuille introduite dans une partie de mise en prise des premier et second rouleaux pour avoir des creux et des saillies ; et
une unité de traitement par ultrasons comportant une machine de soudure par ultrasons et étant configurée pour recouvrir la seconde feuille avec la première feuille déformée ayant des saillies et des creux et pincer les première et seconde feuilles recouvertes entre les saillies du premier rouleau et un émetteur d'ultrasons de la machine de soudage par ultrasons pour appliquer partiellement des vibrations ultrasonores aux feuilles et former les soudures par fusion ayant des trous traversants.

9. Appareil pour fabriquer une feuille composite selon la revendication 8, dans lequel l'appareil comprend en outre un moyen pour chauffer l'émetteur d'ultrasons.

10. Appareil pour fabriquer une feuille composite selon la revendication 8 ou 9, dans lequel l'appareil comprend en outre un dispositif de préchauffage pour préchauffer au moins une parmi la première feuille et la seconde feuille jusqu'à une température prédéterminée avant une application des vibrations ultrasonores.

11. Appareil pour fabriquer une feuille composite selon la revendication 10, dans lequel le dispositif de préchauffage est un dispositif de chauffage disposé dans le premier rouleau.

12. Appareil pour fabriquer une feuille composite selon l'une quelconque des revendications 8 à 11, dans lequel un élément ayant une propriété de stockage de chaleur est disposé au niveau d'une partie d'extrémité distale de l'émetteur d'ultrasons.

13. Appareil pour fabriquer une feuille composite selon la revendication 12, dans lequel l'élément ayant une propriété de stockage de chaleur a une conductivité thermique inférieure à une conductivité thermique d'un matériau de l'émetteur d'ultrasons.

14. Appareil pour fabriquer une feuille composite selon l'une quelconque des revendications 12 à 13, dans lequel l'élément ayant une propriété de stockage de chaleur comporte du verre ou du polyimide.

15. Appareil pour fabriquer une feuille composite selon l'une quelconque des revendications 12 à 13, dans lequel l'émetteur d'ultrasons comporte, au niveau de la partie d'extrémité distale, une couche de résine synthétique qui est résistante à la chaleur en tant qu'élément ayant une propriété de stockage de chaleur.
